# EUROPEAN PATENT SPECIFICATION

(11) **EP 4 261 539 B1**
(45) Date of publication and mention of the grant of the patent: **11.09.2024**
(21) Application number: 22813388.0
(22) Date of filing: 24.02.2022
(51) Int. Cl.: G01N 33/52

(54) **BLOOD TESTING DEVICE**
BLUTTESTVORRICHTUNG
DISPOSITIF DE TEST SANGUIN

(43) Date of publication of application: 18.10.2023
(73) Proprietor: Cellspect Co., Ltd., Morioka-shi, Iwate 020-0857 (JP)
(72) Inventor: KIM, Seung Kyum, Morioka City, Iwate 0200857 (JP); ITO, Takashi, Morioka City, Iwate 0200857 (JP); EDURA, Tomohiko, Morioka City, Iwate 0200857 (JP)
(74) Representative: Hasegawa, Kan
(86) International application number: PCT/JP2022/007627
(87) International publication number: WO 2023/162095

(56) References cited:
- EP-A1- 1 746 419
- WO-A1-2005/106463
- JP-A- 2001 330 605
- JP-A- 2014 529 083
- JP-A- H04 232 855
- JP-A- H05 273 207
- JP-A- H06 242 107
- JP-A- H10 206 417
- JP-A- H10 206 419
- JP-B2- S6 151 737
- JP-Y2- H0 142 041
- US-A- 4 477 575
- US-A1- 2006 062 688
- US-A1- 2011 155 590
- US-A1- 2015 299 760

## Description

### TECHNICAL FIELD

The present invention relates to a blood test device for measuring the presence or concentration of specific substances in the blood.

### BACKGROUND ART

Blood tests are widely used to analyze the presence or concentration of specific substances in the blood in order to diagnose the health of the subject. For example, by analyzing the content of various components in the plasma in the blood, it is possible to diagnose the organ function of the subject.

In general blood tests, the blood, which is the sample, is separated into plasma or serum, which is a liquid component, and cells or the like, which are solid components, by using centrifuges or suction/pressure pumps. Typically, a certain amount of blood (e.g., 5 mL or more) is required for centrifugation, so physicians, nurses, or clinical laboratory technicians need to collect blood from the subject. Therefore, general blood tests can only be performed at medical institutions or specialized test institutions equipped with expensive analytical instruments, such as centrifuges, and power supply facilities to operate them. In addition, there is also a heavy burden on medical professionals who collect blood. In effect, in general blood tests, the burden is high on not only the subject but also the medical institutions and the cost is high as well.

Meanwhile, there is a growing awareness in society that people should manage their own health from the perspective of preventive medicine and the extension of healthy life expectancy. Along with this, there is an increasing need for simple test devices that can perform tests in a rapid manner and the results thereof can be determined visually.

If there is such a simple test device that can separate plasma or serum from blood without using expensive analytical equipment or power supplies, and can further test the presence or concentration of specific substances contained in the separated plasma or serum with high accuracy, people without expertise can perform the test by themselves using a minute amount of self-collected blood even if they are not at well-equipped medical institutions. Therefore, such test will be of great help not only for the daily health management of ordinary people, but also for the health management of people at home who have difficulty going out. Moreover, such test can be conducted in countries and/or regions with vulnerable medical systems, or in areas with power supply difficulties, such as disaster-stricken areas. Accordingly, there is a great demand from society for the development of easy-to-use and accurate test devices.

PLT 1 discloses a reagent strip equipped with: a test pad that contains a coloring reagent system specific to an analyte, and that is formed by an anisotropic film having a side with relatively small holes defining a test surface, and an opposite side with relatively large holes defining a sample receiving surface; and a porous sample transfer medium that is attached to the sample receiving surface of the test pad, the transfer medium being capable of receiving a whole blood sample and transferring a detectable portion of the sample to the sample receiving surface. PTL 2 discloses a sample analysis tool including a blood cell separation layer including a lateral flow filtration filter layer. PTL 3 discloses a composition for permitting the separation of plasma or serum from whole blood, including glass fibers having certain range of diameter and density. Further, PTL 4 describes a bodily fluid analyzer including a test strip holder comprising a base and a snap-on cover, and the test strip comprises a woven dispersement layer, a depth filter containing a reagent, an asymmetrical membrane containing additional reagent, and a colorimetric detection membrane in a vertical stack.

### PRIOR ART DOCUMENTS

### PATENT LITERATURE

PTL 1: JP06-074953 A
PTL 2: EP 1 746 419 A1
PTL 3: US 4 477 575 A
PTL 4: US 2006/061688 A1

### SUMMARY OF THE INVENTION

### PROBLEMS TO BE SOLVED BY THE INVENTION

In a test device in which a plasma separation part composed of a porous member and configured to separate plasma by allowing blood to pass therethrough, and a reagent phase composed of a dry reagent phase in which a reagent is impregnated, are laminated, the plasma separated by the plasma separation part is impregnated from one side of the reagent phase, and a color reaction generated by the plasma reaching the other side of the reagent phase is observed. Such simple test device has the following problems.

In order to obtain reliable and reproducible test results, the entire surface of the reagent phase must be made to make contact with the necessary and sufficient amount of plasma. Because if the amount of plasma is insufficient, there is a risk that the sensitivity of the color reaction caused by the contact of the plasma with the reagent phase may be low and the reaction may not be observable. There will also be a problem of uneven reaction of the reagent phase, resulting in a decrease in the reproducibility of the test. On the other hand, if the amount of plasma is too high, the plasma overflows from the reagent phase, and an inhomogeneous reaction may occur due to the plasma flowing from the side of the reagent phase to the surface. In other words, it is important to supply an appropriate amount of plasma to the reagent phase so that an accurate color reaction according to the concentration of a specific substance in the blood with good reproducibility can be obtained. In order to do so, it is necessary to add blood sample within the appropriate volume range to the test device.

However, for a test device to be used by a general subject himself, simple operation such as self- blood sampling by puncturing a fingertip and directly adding (dripping or spotting) blood onto the test device from the fingertip is expected. It is not practical for the subject to fix the blood volume by using pipettes or other instruments for such test devices.

In addition, in the simple test device described above, plasma evaporation from the surface of the reagent phase results in the color change of the reagent phase after a period of time from the start of the color reaction. Therefore, if colorimetric determination takes time, there will be a problem to the effect that it becomes difficult to obtain stable test results.

The present invention has been made in view of the above, and an object of the present invention is to provide a blood test device that can be used in a simple manner, provides good reliability and reproducibility, and can obtain stable determination results.

### MEANS FOR SOLVING THE PROBLEMS

In order to solve the above-described problems, a blood test device, which is an aspect of the present invention, is a blood test device for measuring the presence or concentration of a specific substance in blood based on a color reaction that occurs in a dry reagent phase impregnated with a reagent by bringing plasma separated from the blood into contact with the dry reagent phase. The blood test device includes: a first substrate with a first opening where the blood is to be added; a second substrate arranged opposite the first substrate, with a second opening to observe the color reaction; the dry reagent phase laminated on the second surface between the first substrate and the second substrate so that a part of the dry reagent phase is exposed from the second opening; a plasma separation part formed by a porous sheet material and laminated on the dry reagent phase; and a diffusion layer formed by a sheet of hydrophilic fibers and arranged on the plasma separation part so that a part of the diffusion layer is exposed from the first opening, wherein the area of a surface of the plasma separation part on the dry reagent phase side, the area being outside the section in contact with the dry reagent phase, is bonded to the second substrate.

In the above-described blood test device, a ratio of the volume of the plasma separation part to the volume of the dry reagent phase may be between 1.6 times and 3.7 times, inclusive.

In the above-described blood test device, a plurality of the second openings may be provided, a plurality of the dry reagent phases may be provided, and the ratio of the volume of the plasma separation part to the sum of the volumes of the dry reagent phases may be between 1.6 times and 3.7 times, inclusive.

In the above-described blood test device, the ratio may be between 2.0 times and 3.1 times, inclusive.

The above-described blood test device may further include a spacer for forming a space with a predetermined height between the first substrate and the second substrate, the spacer being arranged between the first substrate and the second substrate, wherein the dry reagent phase, the plasma separation part, and the diffusion layer may be arranged in the space, and the height of the spacer may be less than the sum of the thickness of the diffusion layer, the thickness of the plasma separation part, and the thickness of the dry reagent phase, in the state before assembly of the blood test device.

In the above-described blood test device, the height of the spacer may be between 0.65 times and 0.9 times the sum, inclusive.

In the above-described blood test device, the plasma separation part may include a polysulfone membrane or an asymmetric polysulfone membrane.

### EFFECT OF THE INVENTION

According to the present invention, a blood test device is achieved that can be used in a simple manner, provides good reliability and reproducibility, and can obtain stable determination results.

### BRIEF DESCRIPTION OF THE DRAWINGS

[FIG. 1] FIG. 1 is a perspective view showing a blood test device according to an embodiment of the present invention (observation part side).
[FIG. 2] FIG. 2 is a perspective view (blood addition part side) of the blood test device according to an embodiment of the present invention.
[FIG. 3] FIG. 3 is an exploded perspective view of the blood test device shown in FIG. 2.
[FIG. 4] FIG. 4 is a partial enlarged sectional view of the blood test device shown in FIG. 2.
[FIG. 5] FIG. 5 is a graph showing the mean values of colorimetric sensitivity in experimental test devices.
[FIG. 6] FIG. 6 is a graph showing the CV values of the colorimetric sensitivity in the experimental test devices.
[FIG. 7] FIG. 7 is an exploded perspective view of a blood test device according to a variation of the embodiment of the present invention.

### EMBODIMENTS OF THE INVENTION

Hereinafter, a blood test device according to embodiments of the present invention will be described with reference to the drawings. It should be noted that the present invention is not limited by these embodiments. In the description of each drawing, the same parts are denoted by the same reference numbers.

The drawings referred to in the following description are merely schematic representations of shape, size, and positional relationship to the extent that the subject matter of the present invention may be understood. In other words, the present invention is not limited only to the shapes, sizes, and positional relationships illustrated in the respective figures. In addition, the drawings may also include, among themselves, parts having different dimensional relationships and ratios from each other.

The phrase "a minute amount" referred to herein refers to the amount between approximately several µL and several tens of µL.

The blood test device described below is for measuring the presence or concentration of a specific substance in plasma based on a color reaction that occurs in a dry reagent phase when the plasma separated from blood comes into contact with the dry reagent phase impregnated with a reagent. The test items that can be measured are components contained in plasma and are not particularly limited as long as they are components that exhibit color reactions to predetermined reagents. Specific examples of test items include minerals such as zinc and magnesium, in addition to AST, ALT, γ-GT, neutral fat, HDL cholesterol, LDL cholesterol, blood sugar, HbA1c, and the like.

### (Configuration of Test Device)

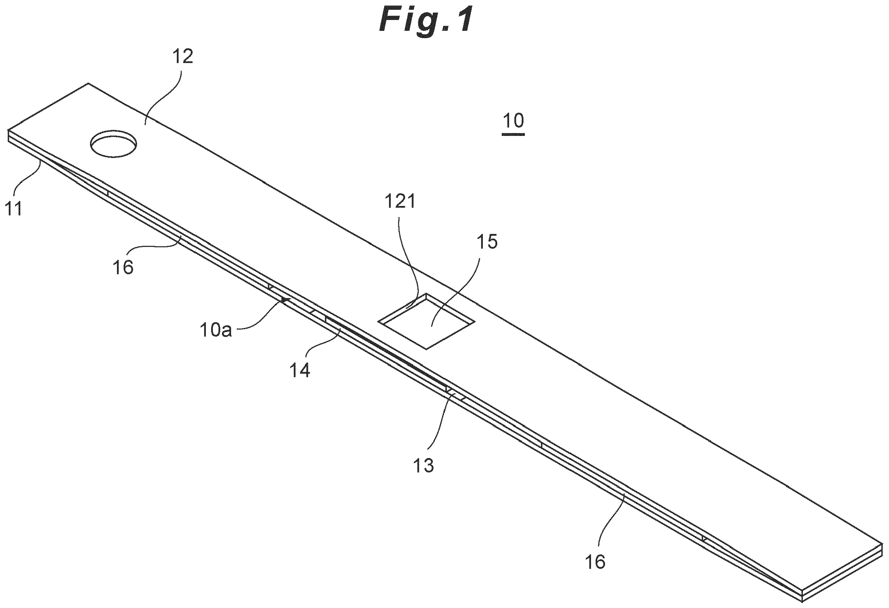

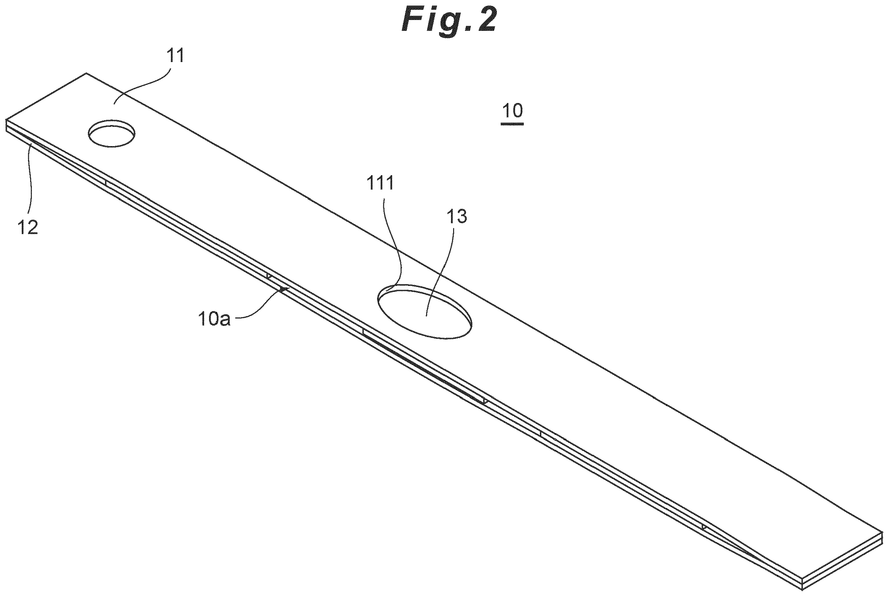

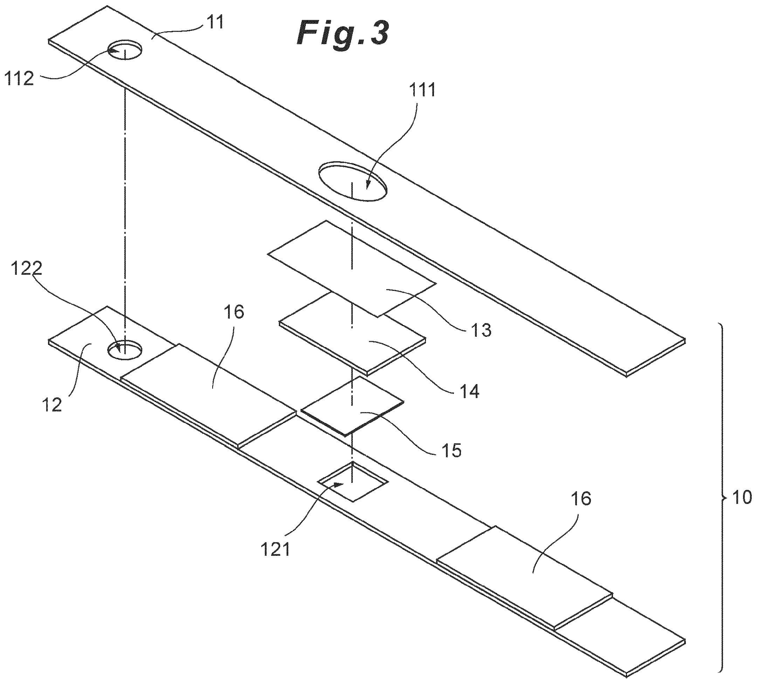

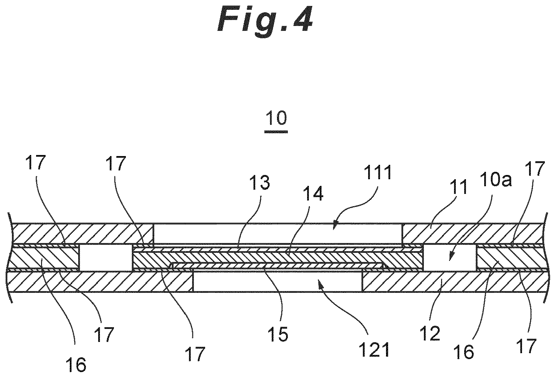

FIGS. 1 and 2 are perspective views showing a blood test device according to a first embodiment of the present invention. FIG. 1 shows the state in which the blood test device is seen from the observation part side, and FIG. 2 shows the state in which the blood test device is seen from the blood addition part side. FIG. 3 is an exploded perspective view of such blood test device. FIG. 4 is a partial enlarged sectional view of such blood test device.

As shown in FIGS. 1 to 4, the blood test device 10 (which may hereinafter be simply referred to as the "test device") according to the present embodiment is equipped with: a first substrate 11 and a second substrate 12, which are arranged opposite each other; and a diffusion layer 13, a plasma separation part 14, and a reagent phase 15, which are each laminated between the first substrate 11 and the second substrate 12.

The first substrate 11 and the second substrate 12 are formed by a thin sheet material made of resin materials such as polyethylene terephthalate (PET). However, the materials of the first substrate 11 and the second substrate 12 are not limited to resins, and any material may be used as long as it can maintain the shape of the test device 10 and is surface-treated so that impregnation of or reaction with blood or plasma, which is the sample, is prevented. For example, the first substrate 11 and the second substrate 12 may be formed by cardboard having a water-repellent surface.

A blood addition part 111, which is an opening into which blood (sample) is added, is formed in the first substrate 11. In the present embodiment, the blood addition part 111 has an elliptical shape, but the shape is not particularly limited if it is a shape that allows the addition of blood. Specific examples include a rectangle, an oval, and the like. An alignment opening 112 may also be formed in the first substrate 11.

An observation part 121, which is an opening for observing the color reaction of the reagent phase 15, is formed in the second substrate 12. In the present embodiment, the observation part 121 has a square shape, but the shape is not particularly limited, and it may be circular, elliptical, or the like. From the viewpoint of easy observation of the color reaction, the shape of the observation part 121 is preferably made similar to the shape of the reagent phase 15, and the area of the reagent phase 15 exposed from the observation part 121 is preferably as large as possible. An alignment opening 122 may also be formed in the second substrate 12.

By providing such first substrate 11 and second substrate 12, the deformation of the diffusion layer 13, the plasma separation part 14, and the reagent phase 15 can be prevented and these three layers can remain in contact with each other. The first substrate 11 and the second substrate 12 may be formed by two plates separated from each other as shown in FIG. 3, or by folding back one elongated plate.

A spacer 16 is arranged between the first substrate 11 and the second substrate 12. The spacer 16 forms a space 10a with a predetermined height between the first substrate 11 and the second substrate 12. In this space 10a, the reagent phase 15, the plasma separation part 14, and the diffusion layer 13 are laminated, in this order, from the second substrate 12 side.

The spacer 16 is formed by a plate which is deform (compression)-resistant to a pressing force in the thickness direction, as with resin materials such as PET, and does not get impregnated with or react with blood or plasma, which are the samples.

As shown in FIG. 4, in the present embodiment, the spacer 16 is bonded to the first substrate 11 and the second substrate 12 by a double-sided tape 17. However, the method of fixing the spacer 16 to the first substrate 11 and the second substrate 12 is not limited to the double-sided tape 17, and a liquid or paste adhesive may be used, or thermal compression bonding may be applied. In short, it is sufficient if the height of the space 10a can be maintained at a constant level by the spacer 16.

The thickness of the spacer 16, i.e., the height of the space 10a, is less than the sum of the thickness of the diffusion layer 13, the thickness of the plasma separation part 14, and the thickness of the reagent phase 15, in the state before the assembly of the test device 10. In other words, in the state where the test device 10 is assembled, the diffusion layer 13, the plasma separation part 14, and the reagent phase 15 are subjected to constant pressure and are maintained in close contact with the adjacent layers by means of the first substrate 11 and the second substrate 12. The thickness of the spacer 16 is preferably between 0.65 times the above sum and 0.9 times the above sum, inclusive, and more preferably between 0.7 times the above sum and 0.85 times the above sum, inclusive. If the thickness of the spacer 16 is too small with respect to the above sum (e.g., less than 0.65 times), the plasma separation part 14 and the reagent phase 15 may become too compressed and clogged. In addition, if the thickness of the spacer 16 is too large with respect to the above sum (e.g., more than 0.9 times), it is difficult to keep the diffusion layer 13, the plasma separation part 14, and the reagent phase 15 in close contact.

The diffusion layer 13 is formed by a member obtained by weaving hydrophilic fibers into a sheet shape, and is arranged so that part of the diffusion layer 13 is exposed from the blood addition part 111 provided on the first substrate 11. The diffusion layer 13 induces the blood added to the blood addition part 111 rapidly in a wide area along the plane direction due to capillary action, and impregnates the blood uniformly on one side of the plasma separation part 14. In the present embodiment, a member obtained by plain weaving hydrophilic polyester is used as the diffusion layer 13. In the present embodiment, the diffusion layer 13 is bonded to the first substrate 11 by the double-sided tape 17 attached around the blood addition part 111. Of course, an adhesive, or the like, may be used, instead of the double-sided tape 17, to bond the diffusion layer 13 to the first substrate 11.

The plasma separation part 14 is formed by a porous sheet material, and traps cell components of the blood impregnated on one side and separates the plasma by allowing liquid components to pass therethrough in the membrane thickness direction. A porous polymer membrane such as a polysulfone (PS) membrane and an asymmetric PS membrane may be used as the plasma separation part 14. In particular, the asymmetric PS membrane has excellent blood separation performance and can provide colorless and transparent plasma without mixing whole blood or hemolysis, even when the thickness of the membrane is as thin as only approximately a few millimeters. In the present embodiment, an asymmetric PS membrane is used as the plasma separation part 14. The asymmetric PS membrane has directionality and is arranged in a direction that allows liquid to pass from the first substrate 11 to the second substrate 12. In addition, a member with a multilayer structure in which a PS membrane, or the like, and a glass fiber membrane are combined may be used as the plasma separation membrane 14.

The plasma separation part 14 is arranged so that a surface (first surface) thereof on the first substrate 11 side is in contact with the diffusion layer 13 and a surface (second surface) thereof on the second substrate 12 side is in contact with the reagent phase 15. In addition, an area of the second surface of the plasma separation part 14, the area being outside the section which is in contact with the reagent phase 15, is bonded to the second substrate 12 by the double-sided tape 17. This enables the seepage of the plasma from the above-described outside area to be suppressed, uneven reaction of the reagent phase 15 due to the seepage of the plasma to be prevented, and the plasma to be supplied efficiently from the plasma separation part 14 to the reagent phase 15.

An adhesive, or the like, may be used instead of the double-sided tape 17 to bond the plasma separation part 14 to the second substrate 12. In either case, unintentional seepage of the plasma from the plasma separation part 14 may be suppressed by making the above-described outside area closely contact the second substrate 12 in order to eliminate the gap between the two.

The reagent phase 15 is a dry reagent phase in which a reagent corresponding to the test item is impregnated into filter paper (e.g., chromatographic paper) and dried, and is arranged so that part of the reagent phase is exposed from the observation part 121. In the present embodiment, the reagent phase 15 is bonded to the second substrate 12 by the double-sided tape 17 attached around the observation part 121. Of course, an adhesive, or the like, may be used instead of the double-sided tape 17.

The reagent phase 15 reacts and exhibits color with respect to a predetermined substance in the plasma that has passed through the plasma separation part 14. This color reaction can be observed visually from the observation part 121 side when the plasma sufficiently sinks into and across the reagent phase 15.

The ratio of the volume of the plasma separation part 14 to the volume of the reagent phase 15 is preferably between 1.6 times and 3.7 times, inclusive, and more preferably between 2.0 times and 3.1 times, inclusive, in the state before the test device 10 is assembled. If this ratio of the volume is too small (e.g., less than 1.6 times), the amount of plasma held in the plasma separation part 14 is reduced relative to the volume of the reagent phase 15, causing the plasma supplied to the reagent phase 15 to deplete quickly. As a result, the drying of the reagent phase 15 progresses, making it difficult to observe a stable color reaction. On the other hand, if the above-described ratio of the volume is too large (e.g., more than 3.7 times), excess plasma will be supplied to the reagent phase 15, resulting in uneven color reactions or liquid leaks in the reagent phase 15, and thus, there is a risk that the reliability and reproducibility of the test results may be reduced.

### (Usage of Test Device)

First, the fingertip, or the like, is punctured to bleed, forming a blood puddle. Next, the blood addition part 111 side of the test device 10 is directed downward, and the blood addition part 111 is made to come into direct contact with the blood puddle formed on the fingertip, or the like. When the entire surface of the reagent phase 15 exposed from the observation part 121 is wet, the test device 10 is moved away from the fingertip, or the like. After a predetermined time has elapsed, the concentration of the test item is estimated by comparing the color development of the reagent phase 15 with the reference color.

In addition, as another usage, a simple liquid collection tool may be used instead of bringing the test device 10 directly into contact with the blood puddle. For example, the tip of the tubular liquid collection tool may be brought into contact with the blood puddle to hold blood, and the tip may be brought into contact with the blood addition part 111 to transfer blood. In this case, it is also possible to additionally add a diluent after the addition of blood.

As explained above, in the test device 10 according to the present embodiment, the diffusion layer 13 is provided in the blood addition part 111, and therefore, when blood is added to the blood addition part 111, blood flows through the diffusion layer 13 and spreads quickly in the plane direction. Therefore, even if blood is only added to a part of the blood addition part 111, the blood can still be quickly and uniformly impregnated in the entire surface of the plasma separation part 14. This allows for almost simultaneous plasma separation to proceed in the plane direction of the plasma separation part 14, and an almost uniform amount of plasma to sink into and across the entire surface of the reagent phase 15 in contact with the plasma separation part 14. As a result, the variation of the reaction in the reagent phase 15 is suppressed, and test results with good reliability and reproducibility can be obtained.

In the present embodiment, the area of the second surface of the plasma separation part 14, such area being outside the section which is in contact with the reagent phase 15, is bonded to the second substrate 12, and the plasma can therefore be contained in a reaction area as well as the plasma separation membrane 14 can be brought into close contact with the reagent phase 13 to facilitate the penetration of plasma into the reagent phase 15. This enables the unintentional seepage of the plasma from the plasma separation part 14 to be suppressed, as well as allowing uneven reaction of the reagent phase 15 to be prevented and the plasma to be supplied efficiently and without waste from the plasma separation part 14 to the reagent phase 15.

In addition, according to the present embodiment, the ratio of the volume of the plasma separation part 14 to the volume of the reagent phase 15 is preferably between 1.6 times and 3.7 times, inclusive, and more preferably between 2.0 times and 3.1 times, inclusive. Therefore, the plasma held in the plasma separation part 14 after being separated from the blood in the plasma separation part 14 is continuously supplied to the reagent phase 15. This means that even if the plasma evaporates from the surface of the reagent phase 15, the plasma is still supplied sequentially from the back of the reagent phase 15, and a stable color reaction can therefore still be observed at the observation part 121 for some time (at least a few minutes to several tens of minutes).

In the present embodiment, the spacer 16 is arranged between the first substrate 11 and the second substrate 12, and therefore, the diffusion layer 13, the plasma separation part 14, and the reagent phase 15 can be arranged in the space 10a in a slightly pressured state. Thereby, these layers are brought into close contact with each other, and the plasma can therefore be supplied uniformly in the plane direction from the plasma separation part 14 to the reagent phase 15. Accordingly, uneven reaction in the reagent phase 15 can be suppressed and the reliability and reproducibility of test results can be improved. In addition, the plasma held in the plasma separation part 14 is continuously supplied to the reagent phase 15 at an almost constant speed by a constant pressure being applied to the plasma separation part 14 and the reagent phase 15, and stable determination results can therefore be obtained.

Further, in the present embodiment, since the volume ratio of the plasma separation part 14 and the reagent phase 15 is set appropriately, an appropriate amount of plasma can be supplied to the reagent phase 15 without accurately measuring the amount of blood added. In other words, reliable and reproducible test results can still be obtained in a stable manner based on a minute amount of blood, even with a simple method in which the blood addition part 111 is brought into direct contact with the blood puddle formed at the fingertip, or the like. Accordingly, even in regions and/or countries, or the like, where testing facilities are not available, it will be possible for subjects to conduct tests by themselves and use the same for health management.

### (Example)

In a device with a configuration similar to that of the test device 10, an experiment was conducted in the following manner to measure the colorimetric sensitivity in the reagent phase by changing the ratio of the volume of the plasma separation part to the volume of the reagent phase.

### 1. Creation of Experimental Test Device

Nine experimental test devices were prepared for each size of a plasma separation part, each test device having a reagent phase, a plasma separation part, and a diffusion layer made of the following materials arranged between two substrates with openings for a blood addition part and an observation part. The thickness of a spacer to keep the clearance between the two substrates was 0.5 mm.

### (1) Reagent phase

Material: Test paper in which a glucose concentration measurement reagent (components: piperazine-1,4-bis(2-ethane sulfonic acid) (PIPES), N-ethyl-N-(2-hydroxy-3-sulfopropyl)-3,5-dimethoxyaniline sodium (DAOS), 4-aminoantipyrine (4-AA), mutarotase, peroxidase (POD), glocose oxidase) is impregnated and dried in chromatographic paper
Size: 5 mm x 5 mm, thickness: 0.17 mm (volume: 4.25 mm³)

### (2) Plasma separation part

Material: asymmetric polysulfone
Six different sizes: 5 mm x 4 mm, 5 mm x 5 mm, 5 mm x 6 mm, 5 mm x 7 mm, 5 mm x 8 mm, 5 mm x 9 mm, and 5 mm x 10 mm, thickness: 0.33 mm only (volume: 6.6 mm³ to 16.5 mm³)

### (3) Diffusion layer

Material: hydrophilic polyester plain woven sheet
Size: 5 mm x 10 mm, thickness: 0.12 mm

### 2. Liquid Sample

10 µL of controlled blood (plasma 55%, blood cells 45%)

### 3. Experimental Method

(1) 10 µL of controlled blood was added to the blood addition part of the test device.
(2) three minutes after the sample addition, the reagent phase exposed in the observation part was captured by an optical camera, and each of the RGB values in the image of the portion of the reagent phase was measured.
(3) after an additional two minutes (i.e., five minutes after the sample addition), the reagent phase exposed in the observation part was captured by the optical camera, and each of the RGB values in the image of the portion of the reagent phase was measured.
(4) the ratio of the RGB values of the reagent phase three minutes after the sample addition to the RGB values of the reagent phase five minutes after the sample addition was calculated (i.e., RGB values after five minutes/RGB values after three minutes). This ratio was used as the colorimetric sensitivity.
(5) mean values and CV values of the above-described colorimetric sensitivity were calculated for each plasma separation part size.

### 4. Experimental Results

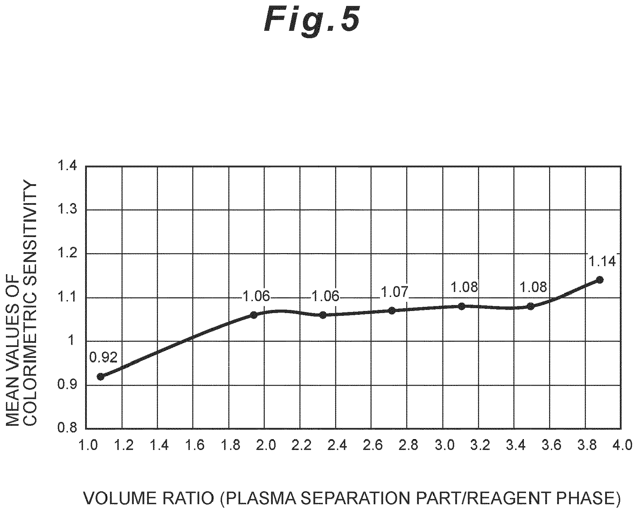

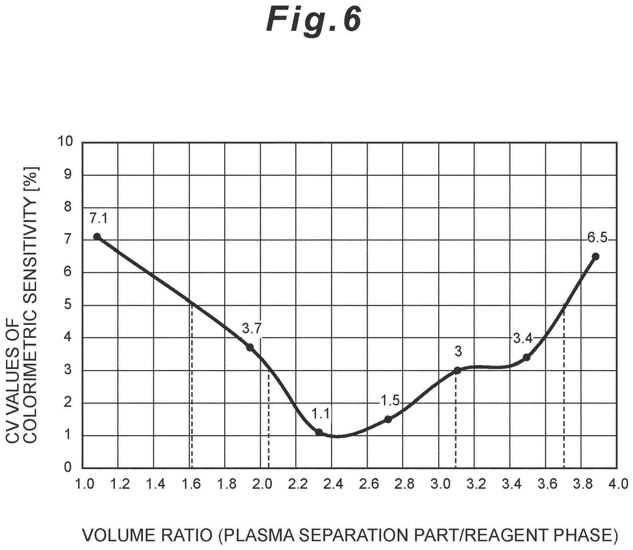

FIG. 5 is a graph showing the mean values of the colorimetric sensitivity (in the case of the R value) in the experimental test devices. FIG. 6 is a graph showing the CV values of the colorimetric sensitivity (in the case of the R value) in the experimental test devices. In FIGS. 5 and 6, the horizontal axis shows the ratio of the volume of the plasma separation part to the volume of the reagent phase.

Here, the colorimetric sensitivity shows that the closer the value to 1, the less the RGB values change even after the passage of time, i.e., the more stable the test results that can be obtained. In addition, the CV values of the colorimetric sensitivity indicate that the lower the value, the less variability of the result, i.e., the higher the reproducibility of the result, and in turn, the higher the reliability of the result.

As shown in FIGS. 5 and 6, when the ratio of the volume is in the range of about 1.6 to 3.7 times, the CV values of the colorimetric sensitivity are relatively less variable, with the CV values being approximately 5% or lower, and the mean values of the colorimetric sensitivity are also within the range of around 1 (+/- 0.1). In addition, when the ratio of the volume is in the range of about 2.0 times to 3.1 times or less, the CV values of colorimetric sensitivity are further relatively less variable, with the CV values being less than 3%.

From the above experimental results, it was found that stable and less variable test results could be obtained by setting the ratio of the volume of the plasma separation part to the volume of the reagent phase between 1.6 times and 3.7 times, inclusive, in the test devices. In addition, it was found that the variation in the test results could be further suppressed by setting the above-described volume ratio between 2.0 times and 3.1 times, inclusive. Here, in this specification, the ratio of the volume of the plasma separation part to the volume of the reagent phase is not strictly limited to the above-described ratio, and may generally include a range of +/- 10%.

### (Variation)

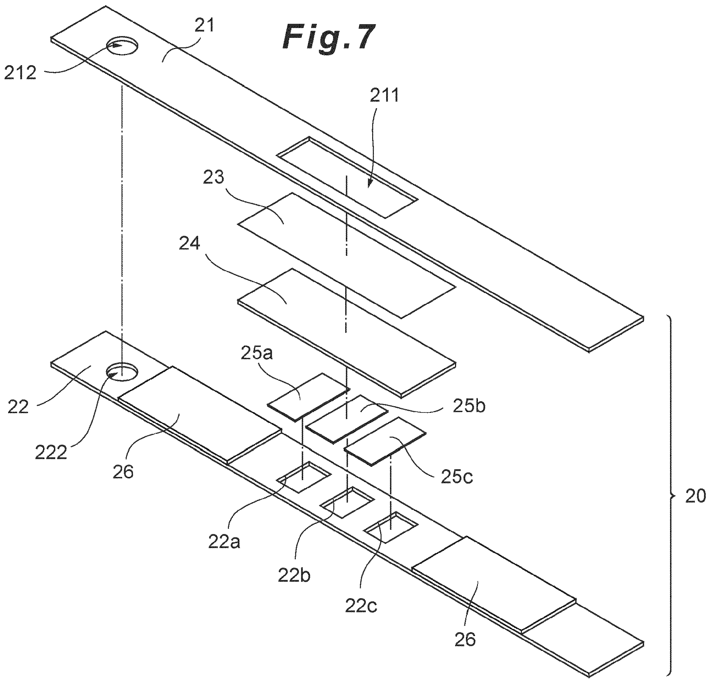

FIG. 7 is an exploded perspective view of a blood test device according to a variation of the embodiment of the present invention. As shown in FIG. 7, the blood test device 20 according to the present variation is a device for testing multiple items and is equipped with: a first substrate 21 and a second substrate 22, which are arranged opposite each other via a spacer 26; and a diffusion layer 23, a plasma separation part 24, and a plurality of reagent phases 25a, 25b, 25c, which are arranged between the first substrate 21 and the second substrate 22. The materials and functions of these parts are similar to those of the first substrate 11, the second substrate 12, the diffusion layer 13, the plasma separation part 14, the reagent phase 15, and the spacer 16, in the first embodiment.

A blood addition part 211, which is an opening into which blood (sample) is added, is formed in the first substrate 21. The outer shape of the blood addition part 211 is not particularly limited, and it may be rectangular, as shown in FIG. 7, or cylindrical, oval, and so on.

In addition, a plurality of observation parts 22a, 22b, 22c, which are openings for observing color reaction, are formed in the second substrate 22. The reagent phases 25a, 25b, and 25c are respectively arranged in such a way that parts of them are exposed from the observation parts 22a, 22b, and 22c. Alignment openings 212, 222 may also be respectively formed in the first and second substrates 21, 22.

An area of the surface of the plasma separation part 24 on the side of the reagent phases 25a, 25b, and 25c, the area excluding the section in contact with the reagent phases 25a, 25b, and 25c, is bonded to the second substrate 22 by double-sided tape, adhesive, or the like. The area also includes the area between the reagent phases 25a, 25b, and 25c. As a result, there is a suppression of leakage of plasma from the area of the surface of the plasma separation part 24 on the side of the reagent phases 25a, 25b, and 25c, such area excluding the section in contact with the reagent phases 25a, 25b, and 25c. In addition, circulation of the liquid through passages between adjacent reagent phases 25a, 25b, and 25c can be prevented, thereby suppressing plasma seepage between adjacent reagent phases. As a result, uneven reactions in the respective reagent phases 25a, 25b, and 25c due to the plasma seepage, and color staining between the adjacent reagent phases 25a, 25b, and 25c can be prevented, and efficient and waste-free supply of plasma to the reagent phases 25a, 25b, and 25c from the plasma separation part 24 can be achieved.

In the present variation, as in the above-described embodiment, the ratio of the volume of the plasma separation part 24 to the sum of the volumes of the multiple reagent phases 25a, 25b, and 25c, is also preferably set between 1.6 times and 3.7 times, inclusive, and more preferably between 2.0 times and 3.1 times, inclusive.

### DESCRIPTION OF REFERENCE NUMBERS

10, 20 ... blood test device (test device), 10a ... space, 11, 21 ... first substrate, 12, 22 ... second substrate, 13, 23 ... diffusion layer, 14, 24 ... plasma separation part, 15, 25a, 25b, 25c ... reagent phase, 16, 26 ... spacer, 17 ... double-sided tape, 111, 211 ... blood addition part, 112, 122, 212, 222 ... opening, 121, 22a, 22b, 22c ... observation part

## Claims

1. A blood test device (10,20) for measuring the presence or concentration of a specific substance in blood based on a color reaction that occurs in a dry reagent phase impregnated with a reagent by bringing plasma separated from the blood into contact with the dry reagent phase, comprising:
a first substrate (11, 21) with the first opening (111, 211) where the blood is to be added;
a second substrate (12, 22) arranged opposite the first substrate (11, 21), with a second opening (121, 22a, 22b, 22c) to observe the color reaction;
the dry reagent phase (15, 25a, 25b, 25c) laminated on the second surface between the first substrate (11, 21) and the second substrate so that a part of the dry reagent phase (15, 25a, 25b, 25c) is exposed from the second opening (121, 22a, 22b, 22c);
a plasma separation part (14, 24) formed by a porous sheet material and laminated on the dry reagent phase (15, 25a, 25b, 25c); and
a diffusion layer (13, 23) formed by a sheet of hydrophilic fibers and arranged on the plasma separation part (14, 24) so that a part of the diffusion layer (13, 23) is exposed from the first opening (111, 211),
wherein the area of a surface of the plasma separation part (14, 24) on the dry reagent phase side, the area being outside the section in contact with the dry reagent phase (15, 25a, 25b, 25c), is bonded to the second substrate (12, 22).

2. The blood test device (10,20) according to claim 1, wherein a ratio of the volume of the plasma separation part (14, 24) to the volume of the dry reagent phase (15, 25a, 25b, 25c) is between 1.6 times and 3.7 times, inclusive.

3. The blood test device (20) according to claim 1, wherein
a plurality of the second openings (22a, 22b, 22c) is provided,
a plurality of the dry reagent phases (25a, 25b, 25c) is provided, and
the ratio of the volume of the plasma separation part (14, 24) to the sum of the volumes of the dry reagent phases (25a, 25b, 25c) is between 1.6 times and 3.7 times, inclusive.

4. The blood test device (10,20) according to claim 2 or 3, wherein the ratio is between 2.0 times and 3.1 times, inclusive.

5. The blood test device (10,20) according to any one of claims 1 to 4, further comprising a spacer for forming a space with a predetermined height between the first substrate (11, 21) and the second substrate (12, 22), the spacer being arranged between the first substrate (11, 21) and the second substrate (12, 22),
wherein the dry reagent phase (15, 25a, 25b, 25c), the plasma separation part (14, 24), and the diffusion layer (13, 23) are arranged in the space, and
the height of the spacer is less than the sum of the thickness of the diffusion layer (13, 23), the thickness of the plasma separation part (14, 24), and the thickness of the dry reagent phase (15, 25a, 25b, 25c), in the state before assembly of the blood test device.

6. The blood test device (10,20) according to claim 5, wherein the height of the spacer is between 0.65 times and 0.9 times the sum, inclusive.

7. The blood test device (10,20) according to any one of claims 1 to 6, wherein the plasma separation part (14, 24) includes a polysulfone membrane or an asymmetric polysulfone membrane.

## Patentansprüche

1. Bluttestvorrichtung (10,20) zum Messen des Vorhandenseins oder der Konzentration einer spezifischen Substanz im Blut basierend auf einer Farbreaktion, die in einer mit einem Reagens getränkten trockenen Reagensphase auftritt, indem aus dem Blut abgetrenntes Plasma mit der trockenen Reagensphase in Kontakt gebracht wird, umfassend:
ein erstes Substrat (11, 21) mit der ersten Öffnung (111, 211), wo das Blut hinzuzufügen ist;
ein zweites Substrat (12, 22), das gegenüber dem ersten Substrat (11, 21) angeordnet ist, mit einer zweiten Öffnung (121, 22a, 22b, 22c) zum Beobachten der Farbreaktion;
die trockene Reagensphase (15, 25a, 25b, 25c), die auf der zweiten Oberfläche zwischen dem ersten Substrat (11, 21) und dem zweiten Substrat laminiert ist, sodass ein Teil der trockenen Reagensphase (15, 25a, 25b, 25c) aus der zweiten Öffnung (121, 22a, 22b, 22c) freigelegt ist;
einen Plasmatrennteil (14, 24), der aus einem porösen Bahnmaterial ausgebildet ist und auf die trockene Reagensphase (15, 25a, 25b, 25c) laminiert ist; und
eine Diffusionsschicht (13, 23), die aus einer Bahn aus hydrophilen Fasern ausgebildet ist und so auf dem Plasmatrennteil (14, 24) angeordnet ist, dass ein Teil der Diffusionsschicht (13, 23) aus der ersten Öffnung (111, 211) freigelegt ist,
wobei der Bereich einer Oberfläche des Plasmatrennteils (14, 24) auf der Seite der trockenen Reagensphase, wobei sich der Bereich außerhalb des Abschnitts befindet, der mit der trockenen Reagensphase (15, 25a, 25b, 25c) in Kontakt steht, an das zweite Substrat (12, 22) gebunden ist.

2. Bluttestvorrichtung (10,20) nach Anspruch 1, wobei ein Verhältnis des Volumens des Plasmatrennteils (14, 24) zu dem Volumen der trockenen Reagensphase (15, 25a, 25b, 25c) zwischen 1,6-mal und einschließlich 3,7-mal liegt.

3. Bluttestvorrichtung (20) nach Anspruch 1, wobei
eine Vielzahl der zweiten Öffnungen (22a, 22b, 22c) bereitgestellt ist,
eine Vielzahl der trockenen Reagensphasen (25a, 25b, 25c) bereitgestellt ist, und
das Verhältnis des Volumens des Plasmatrennteils (14, 24) zu der Summe der Volumen der trockenen Reagensphasen (25a, 25b, 25c) zwischen 1,6-mal und einschließlich 3,7-mal liegt.

4. Bluttestvorrichtung (10,20) nach Anspruch 2 oder 3, wobei das Verhältnis zwischen 2,0-mal und einschließlich 3,1-mal liegt.

5. Bluttestvorrichtung (10,20) nach einem der Ansprüche 1 bis 4, ferner einen Abstandshalter zum Ausbilden eines Raums mit einer vorbestimmten Höhe zwischen dem ersten Substrat (11, 21) und dem zweiten Substrat (12, 22) umfassend, wobei der Abstandshalter zwischen dem ersten Substrat (11, 21) und dem zweiten Substrat (12, 22) angeordnet ist,
wobei die trockene Reagensphase (15, 25a, 25b, 25c), der Plasmatrennteil (14, 24) und die Diffusionsschicht (13, 23) in dem Raum angeordnet sind, und
die Höhe des Abstandshalters geringer als die Summe der Dicke der Diffusionsschicht (13, 23), der Dicke des Plasmatrennteils (14, 24) und der Dicke der trockenen Reagensphase (15, 25a, 25b, 25c) im Zustand vor einem Zusammenbau der Bluttestvorrichtung ist.

6. Bluttestvorrichtung (10,20) nach Anspruch 5, wobei die Höhe des Abstandshalters zwischen 0,65-mal und einschließlich 0,9-mal die Summe liegt.

7. Bluttestvorrichtung (10,20) nach einem der Ansprüche 1 bis 6, wobei der Plasmatrennteil (14, 24) eine Polysulfonmembran oder eine asymmetrische Polysulfonmembran enthält.

## Revendications

1. Dispositif de test sanguin (10, 20) permettant de mesurer la présence ou la concentration d'une substance spécifique dans le sang sur la base d'une réaction colorée qui se produit dans une phase réactive sèche imprégnée d'un réactif en mettant le plasma séparé du sang en contact avec la phase réactive sèche, comprenant :
un premier substrat (11, 21) doté de la première ouverture (111, 211) dans laquelle le sang doit être ajouté ;
un second substrat (12, 22) disposé à l'opposé du premier substrat (11, 21), doté d'une seconde ouverture (121, 22a, 22b, 22c) destinée à observer la réaction colorée ;
la phase réactive sèche (15, 25a, 25b, 25c) stratifiée sur la seconde surface entre le premier substrat (11, 21) et le second substrat de sorte qu'une partie de la phase réactive sèche (15, 25a, 25b, 25c) soit exposée depuis la seconde ouverture (121, 22a, 22b, 22c) ;
une partie de séparation de plasma (14, 24) constituée d'un matériau en feuille poreux et stratifiée sur la phase réactive sèche (15, 25a, 25b, 25c) ; et
une couche de diffusion (13, 23) constituée d'une feuille de fibres hydrophiles et disposée sur la partie de séparation de plasma (14, 24) de sorte qu'une partie de la couche de diffusion (13, 23) soit exposée depuis la première ouverture (111, 211),
la zone d'une surface de la partie de séparation de plasma (14, 24) du côté de la phase réactive sèche, la zone se trouvant à l'extérieur de la section en contact avec la phase réactive sèche (15, 25a, 25b, 25c), étant liée au second substrat (12, 22).

2. Dispositif de test sanguin (10, 20) selon la revendication 1, le rapport du volume de la partie de séparation de plasma (14, 24) au volume de la phase réactive sèche (15, 25a, 25b, 25c) étant compris entre 1,6 fois et 3,7 fois inclus.

3. Dispositif de test sanguin (20) selon la revendication 1,
une pluralité de secondes ouvertures (22a, 22b, 22c) étant pourvue,
une pluralité de phases réactives sèches (25a, 25b, 25c) étant pourvue, et
le rapport du volume de la partie de séparation de plasma (14, 24) à la somme des volumes des phases réactives sèches (25a, 25b, 25c) étant compris entre 1,6 fois et 3,7 fois inclus.

4. Dispositif de test sanguin (10, 20) selon la revendication 2 ou 3, ledit rapport étant compris entre 2,0 fois et 3,1 fois inclus.

5. Dispositif de test sanguin (10, 20) selon l'une quelconque des revendications 1 à 4, comprenant en outre un espaceur permettant de former un espace d'une hauteur prédéfinie entre le premier substrat (11, 21) et le second substrat (12, 22), l'espaceur étant disposé entre le premier substrat (11, 21) et le second substrat (12, 22),
ladite phase réactive sèche (15, 25a, 25b, 25c), ladite partie de séparation de plasma (14, 24) et lesdites couches de diffusion (13, 23) étant disposées dans l'espace, et
ladite hauteur de l'espaceur étant inférieure à la somme de l'épaisseur de la couche de diffusion (13, 23), de l'épaisseur de la partie de séparation de plasma (14, 24) et de l'épaisseur de la phase réactive sèche (15, 25a, 25b, 25c), dans l'état avant montage du dispositif de test sanguin.

6. Dispositif de test sanguin (10, 20) selon la revendication 5, ladite hauteur de l'espaceur étant comprise entre 0,65 fois et 0,9 fois la somme, inclus.

7. Dispositif de test sanguin (10, 20) selon l'une quelconque des revendications 1 à 6, ladite partie de séparation de plasma (14, 24) comprenant une membrane en polysulfone ou une membrane en polysulfone asymétrique.
